# EUROPEAN PATENT APPLICATION

(11) **EP 1 500 386 A1**
(43) Date of publication of application: **26.01.2005**
(21) Application number: 04014656.5
(22) Date of filing: 23.06.2004
(51) Int. Cl.: A61K 7/09

(54) **Method for time-dependent decrease of ph in a cosmetic composition and a composition for permanent hair shaping with time-dependent decrease of the wave-shaping efficiency**

(30) Priority: 23.07.2003 US 489449 P
(71) Applicant: Wella Aktiengesellschaft, 64274 Darmstadt (DE)
(72) Inventor: Rozzell, David, Dr., Burbank, CA 91506 (US); Allwohn, Jürgen, Dr., 65558 Burgschwalbach (DE); Cassier, Thorsten, Dr., 64807 Dieburg (DE)

(57) **Abstract**

A method for time dependent decrease of pH in a cosmetic composition for treating skin or hair, preferably a method for permanently shaping hair, for which, immediately before use, an enzyme that catalyzes an acid-generating reaction with a non-ester substrate to form a ready-to-apply cosmetic composition is added immediately (preferably 5 seconds to 3 minutes) prior to use to an alkaline composition having an initial pH of 7.0 to 14 and comprising a non-ester substrate,. Than the hair, before and/or after it is held in the desired shape, is treated with the ready-to-use agent and, after the period of action, is rinsed with water. Thereafter the hair is treated oxidatively, rinsed again with water, optionally laid into a water wave and then dried. Preferably the enzyme and the non-ester substrate is selected so that the initial pH of 7.0 to 12 is lowered within a time interval of from 5 to 40 minutes by at least 0.5 pH units.

## Description

The present invention relates to a method for time dependent decrease of pH in a cosmetic composition and an agent for permanently shaping hair on the basis of a keratin-reducing active ingredient, an enzyme that catalyzes an acid-generating reaction with a substrate and a substrate. The agent for permanently shaping hair shows a time-dependent gradual reduction in the waving effectiveness and a gentle shaping of hair with a high certainty of the result and improved care properties.

### Description of the Related Art

In general, the process of permanently shaping hair consists of two steps. Initially, the cystine disulfide bonds of the hair keratin are opened reductively by the action of an agent which contains a keratin-reducing active ingredient. A transformation in which smooth hair is waved or curly hair is straightened, becomes possible as a result in that, after the reduction, the hair is rinsed and the cystine disulfide bonds are reestablished in their new position using an agent containing an oxidizing active ingredient.

As active reducing agents, mercaptocarboxylic acids are often used. In particular, thioglycolic acid or thiolactic acid or their salts, mercaptocarboxylate esters, cysteine and its derivatives or cysteamine and its derivates are especially useful.

The compositions containing thioglycolate are used usually at a pH between 8 and 10, and more preferably between a pH of 8.5 and 9.5. If this is done repeatedly, even at short intervals of time, the hair may be damaged.

A higher pH-value leads to an increased swelling of the hair as well as an increase in the reducing power of the mercapto compound used, leading to an increase in the effectiveness of the reducing step. Aside from the usual alkalizing agents, ammonia, ammonium hydrogen carbonate, ammonium carbonate, ammonium carbamate, monoethanolamine and diethanolamine are most commonly used for adjusting the pH to the alkaline range.

The above-mentioned mildly alkaline permanent waving preparations are applied in the usual manner on the hair. To achieve a permanent hair transformation, the agent must act for a certain period of time. Depending on the state of the treated hair, a prolonged period of action or a repeated application of the permanent waving preparation represents an increasing stress in the sense of an irreversible damage to the hair structure. In addition, it is a particular difficulty that the reducing process and the therewith associated softening of the hair proceed even after the prescribed period of action is reached, so that it is very important for the hairdresser to fix the correct end point. This is seen to be particularly relevant in the case of long hair, which has already been previously treated repeatedly with permanent waving agents, oxidative dyeing agents and/or bleaching agents. Since the treatment methods mentioned adversely affect the hair structure but damaged hair is particularly permeable for the shaping agents, excessive curling can result already when the optimum period of action is exceeded relatively slightly.

This problem, which occurs time and again in the daily practice of the hairdresser, has led to a series of attempted solutions, which are intended to counter the danger of damage to the hair during the permanent waving process by decreasing the waving effectiveness of the reducing agent during the period of action.

From our own German patent application DE 23 49 050 A an agent and a method are known for which the waving activity and consequential damage to the hair are decreased continuously by lowering the initial pH. Organic compounds with ester or halogen groups in the molecule, which can be split by alkalis and are capable of forming acid by the splitting, are added to the shaping agent shortly before use. By these means, the concentration of alkali is to decrease gradually and to the desired extent during the period of action. Ethyl acetate and triacetin have been named as examples of suitable esters. In the case of compounds containing ester groups, the splitting can be accelerated by also adding the lipase pancreatin.

Aside from the disadvantageous toxicology and skin compatibility of many of the substances mentioned, the esters also are too unstable in the presence of the shaping solution to be formulated with it. In addition, the low saponification rates of the esters recommended there result in an insufficient release of acid during the period of action of 5 to 30 minutes. The enzyme pancreatin, mentioned in DE 23 49 050 A, accelerates the acid-generation from ester substrates, but pancreatin is insufficient stable and loses activity rapidly in the alkaline reducing solution. This is a significant disadvantage of the method of the prior art. When ester-splitting enzymes like pancreatin are used, the stability problems make it necessary, for instance, to formulate the shaping agent in three components.

An important advantage of the compositions and methods of the present invention is that a non-ester substrate is used. Such non-ester substrates are much more stable than ester-substrates in the presence of alkaline cosmetic compositions as described herein and undergo an acid-generating reaction only in the presence of a specific enzyme. A further advantage of the compositions and methods of the present invention is in the use of an enzyme that is more stable kept separately or even when contained in the alkaline composition than the ester-splitting enzymes as described in DE 2349050 A. In a preferred embodiment a hydantoinase is used as the enzyme. The hydatoinase that is the subject of the present invention has greater stability than ester-splitting enzymes such as pancreatin that are described in the prior art, especially in the presence of alkaline reducing solutions such as those used in the permanent shaping of hair.

### Summary of the Invention

It is therefore an object of the invention to make available a cosmetic composition that comprises an enzyme that catalyzes an acid-generating reaction with a non-ester substrate, a method and an agent for the permanent shaping of hair, for which the pH drops rapidly enough during the period of action, so that the reducing power of the waving agent is weakened during the period of action and, by these means, on the one hand, the hair structure is treated more gently but, on the other, sufficient transformation of the hair is nevertheless achieved. When the pH drops during the period of action the hair damage is reduced due to the decrease of the hair swelling and the weakened reducing power of the waving agent.

It has now surprisingly been found that the above-mentioned disadvantages of the method of the state of the art can be avoided by proceeding according to the present invention.

It is therefore an object of the invention to provide a cosmetic composition that comprises an enzyme that catalyzes an acid-generating reaction with a non-ester substrate.

As used herein, by "cosmetic composition" is meant a solution, a creme, a paste, an ointment or a suspension containing various ingredients and additives typically used in formulating a composition that is applied to the skin and/or the hair. Such ingredients and additives may include, but are not limited to, for example thickening agents, such as bentonite, fatty acids, starch, polyacrylic acid and its derivatives, cellulose derivatives, alginates, Vaseline, paraffin oils, wetting agents or emulsifiers from the classes of anionic, cationic, amphoteric or nonionic surface-active substances, such as fatty alcohol sulfates, fatty alcohol ether sulfates, alkylsulfonates, alkylbenzenesulfates, quaternary ammonium salts, alkylbetaines, ethoxylated alkylphenols, fatty acid alkanolamides or ethoxylated fatty esters, furthermore opacifiers, such as polyethylene glycol esters, alcohols, such as ethanol, propanol, isopropanol, polyols, such as ethylene glycol, 1,2- or 1,3-dihydroxy-propane, 1,2-, 1,3- or 1,4-dihydroxy-butane, 1,2-, 1,3-, 1,4-or 1,5-dihydroxy-pentane and glycerin, sugars, such as D-glucose, solubilizers, stabilizers, buffering substances, perfume oils, dyes as well as hair conditioning and hair caring components, such as cationic polymers, lanolin, lanolin derivatives, cholesterol, pantothenic acid and betaine.

A further object of the invention is a cosmetic composition comprising an enzyme that catalyzes an acid-generating reaction with a non-ester substrate and further comprising the said non-ester substrate.

In a preferred embodiment said enzyme has the property that it shows a half-life in the presence of 10% by weight thioglycolate at pH 8.5 of greater than 30 min.

In a preferred embodiment said non-ester substrate is selected from the group consisting of a compounds represented by formula (I) and (II) 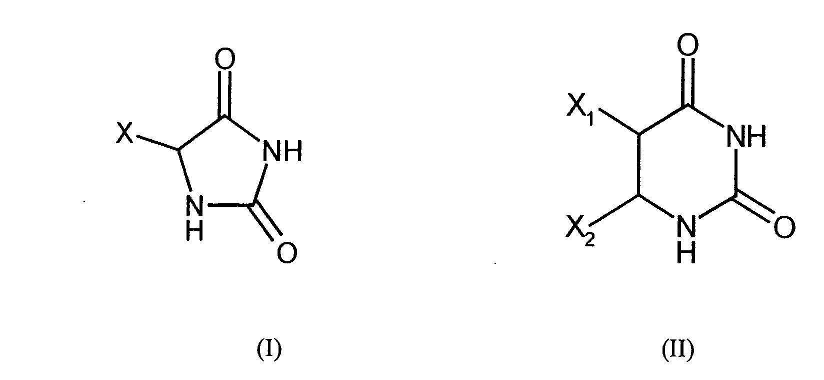 wherein X, X₁ and X₂ are independently selected from the group consisting of hydrogen, alkyl, aryl, arylalkyl and heterocyclic.

As used herein, by "alkyl" is meant a straight chain or branched chain alkyl group having from 1 to 8 carbon atoms and in which 1 to 3 of the carbon atoms may optionally bear substituents selected from the group consisting of hydroxyl, alkoxyl, amino, dialkylamino and carboxyl.

As used herein, by "alkoxyl" is meant an oxygen atom bound to an alkyl group as defined above.

As used herein, by "dialkylamino" is meant an nitrogen atom bound to two alkyl group as defined above.

As used herein, by "aryl" is meant an aromatic ring containing 5 or 6 ring atoms which may be any combination of carbon, oxygen and nitrogen atoms, and which optionally may further contain from 1 to 3 substituents selected from the group consisting of hydroxyl, alkoxyl, amino, dialkylamino and carboxyl.

As used herein, by "heterocyclic" is meant a carbon atom ring having 5 to 7 ring atoms further containing one or more atoms selected from the group consisting of oxygen, nitrogen and sulphur, and which optionally may further contain from 1 to 3 substituents selected from the group consisting of hydroxyl, alkoxyl, amino, dialkylamino and carboxyl.

A further object of the invention is a method for time dependent decrease of pH in a cosmetic composition for treating skin or hair, said method comprising the steps of:
(i) adding an enzyme that catalyzes an acid-generating reaction with a non-ester substrate to an alkaline composition having an initial pH of from about 7.0 to about 14 and comprising said non-ester substrate to form a ready-to-apply cosmetic composition;
(ii) treating the skin or hair of a person with said ready-to-apply cosmetic composition for a time sufficient to achieve the desired cosmetic effect.

In a preferred embodiment the enzyme and the substrate is selected so that the initial pH is from about 7.0 to about 12 and is lowered within a time interval of from about 5 to about 40 minutes by at least about 0.5 pH units.

In another preferred embodiment of the composition and the method as described above the enzyme has the property that it shows a half-life in the presence of 10% by weight thioglycolate at pH 8.5 of greater than 30 minutes.

In a further preferred embodiment of the method the adding of the enzyme is carried out immediately, within 10 minutes, and preferably within 10 seconds to 3 minutes prior to use of the ready-to-apply cosmetic composition.

The method is preferably applied for time dependent decrease of pH for a permanent hair shaping composition.

Thus another object of the invention is a method of permanently shaping hair, said method comprising the steps of:
(a) adding an enzyme that catalyzes an acid-generating reaction with a non-ester substrate to said alkaline permanent shaping composition having an initial pH of from about 7.0 to about 14 and comprising a keratin-reducing active agent and said non-ester substrate being added immediately prior to treating the hair with said shaping agent, to form a ready-to-apply alkaline shaping agent;
(b) putting the hair in a desired shape;
(c) treating the hair with said ready-to-apply shaping agent for a period of action sufficient for permanent shaping of hair;
(d) after the period of action of said shaping agent, optionally rinsing the hair with water;
(e) after rinsing of the hair in step (d) oxidatively treating the hair and then optionally again rinsing the hair with water; and
(f) subsequently optionally putting the hair in a water wave and drying the hair.

Preferably the enzyme and the substrate is selected so that the initial pH is from about 7.0 to about 12 and is lowered within a time interval of from about 5 to about 40 minutes by at least about 0.5 pH units.

Preferably, the initial pH of the alkaline permanent shaping agent is from about 7.5 to about 10.0, more preferably from about 7.8 to about 9.5, and is decreased within about 10 to 30 minutes by about 0.5 to 3.0 units.
More preferably, the initial pH of the alkaline permanent shaping agent is from about 8.0 to about 10,0, and is decreased within about 10 to 30 minutes by about 1.0 to 2.0 units.

It is especially preferred if the initial pH of the permanent shaping agent is from 8,0 to 9,0 and is decreased within 7 to 20 minutes by at least one pH unit to a pH of between 6.5 and 7.3. The application temperature preferably is 20°C to 45°C and more preferably 30°C to 40°C.

In another preferred embodiment of the method the enzyme has the property that it shows a half-life in the presence of 10% by weight thioglycolate at pH 8.5 of greater than 30 min.

The said enzyme that catalyzes an acid-generating reaction with a non-ester substrate is preferably hydantoinase.

For carrying out the inventive method, it has proven to be particularly advantageous to use the enzyme in a concentration of 10 mg/l to 500 mg/l (0.01 to 0.50 % by weight), and preferably of 20 mg/l to 200 mg/l (0.02 to 0.20 % by weight) based on the ready-for-use permanent shaping agent.

The enzyme may be present in pure form or formulated as a mixture together with inert additives, such as perfume oils, wetting agents, solubilizers, dyes, etc., which are usually used in cosmetics. Preferably, the enzyme is dissolved in a nonaqueous solvent, optionally containing a multihydric alcohol such as glycerin. In one preferred embodiment the enzyme is present in an aqueous ammonium sulfate suspension.

The said non ester substrate is preferably selected from the groups consisting of a hydantoin and a dihydrouracil, having the structures represented by formulas (I) or (II).

It has proven to be particularly advantageous to use the substrate in a concentration of 10 g/l to 100 g/l (1 to 10 % by weight) and preferably of 20 g/l to 50 g/l (2 to 5 % by weight), based on the ready-for-use permanent shaping agent.

In a further preferred embodiment of the method the adding of the enzyme is carried out immediately, within 10 minutes, and preferably within 10 seconds to 3 minutes prior to use of the ready-to-apply alkaline shaping agent.

The ready-to-apply permanent hair shaping agent may contain a substance to adjust the pH-value. By suitably selecting the concentration of the buffers, ammonium hydrogen carbonate, ammonium carbonate and/or ammonium carbamate, the pH of the permanent hair shaping agent and the amount of substrate and enzyme added, the decrease in the degree of dissociation of the reducing agent and, with that, the waving effectiveness during the period of action can be controlled to the desired degree by the decrease in the alkalinity. Preferably, a buffer, effective at a pH ranging from 6.8 to 7.5, is also added immediately before use. Aside from increasing the waving strength, the substances ammonium hydrogen carbonate, ammonium carbonate and ammonium carbamate have the task of buffering the pH towards the end of the period of action within the range of 6 to 7. Generally, the amount of buffer used ranges from 0.1 to 15% by weight and preferably from 1 to 8% by weight of the ready-for-use agent for permanently shaping hair.

The acid-generating reaction of the enzyme with the substrate can be accelerated by the heat usually supplied during permanent waving treatments.

As active, keratin-reducing agents, especially thioglycolic acid, thioglycolamide, thioglycerin, thiolactic acid, 3-mercaptopropionic acid, 2-mercaptopropionic acid, 3-mercaptopropionamid, 2-mercaptopropionamid, 2-mercaptoacetamid, cystein, cysteamine, homocysteine, alkyl or acyl cysteines, or the salts or the derivatives of these compounds, especially ammonium thioglycolate, can be used. This active keratin-reducing agent is contained in the ready-for-use agent for the permanent shaping of hair in an amount of 1 to 25% by weight and preferably of 5 to 15% by weight.

Of course, the ready-for-use agent for the permanent shaping of hair may contain all additives, which are customary and known for the permanent shaping, for example, thickening agents, such as bentonite, fatty acids, starch, polyacrylic acid and its derivatives, cellulose derivatives, alginates, Vaseline, paraffin oils, wetting agents or emulsifiers from the classes of anionic, cationic, amphoteric or nonionic surface-active substances, such as fatty alcohol sulfates, fatty alcohol ether sulfates, alkylsulfonates, alkylbenzenesulfates, quaternary ammonium salts, alkylbetaines, ethoxylated alkylphenols, fatty acid alkanolamides or ethoxylated fatty esters, furthermore opacifiers, such as polyethylene glycol esters, alcohols, such as alcohols such as ethanol, propanol, isopropanol, polyols, such as ethylene glycol, 1,2- or 1,3-dihydroxy-propane, 1,2-, 1,3- or 1,4-dihydroxy-butane, 1,2-, 1,3-, 1,4- or 1,5-dihydroxy-pentane and glycerin, sugars, such as D-glucose, solubilizers, stabilizers, buffering substances, perfume oils, dyes as well as hair conditioning and hair caring components, such as cationic polymers, lanolin, lanolin derivatives, cholesterol, pantothenic acid and betaine.

The components mentioned are used in the ready-for-use agent for the permanent shaping of hair in amounts customary for such purposes. For example, the wetting agents and emulsifiers are used in concentrations totaling 0.2 to 30% by weight, the alcohols in an amount totaling 0.1 to 20% by weight, the opacifiers, perfume oils and dyes in an amount of in each case 0.1 to 1% by weight, the buffering substances in an amount totaling 0.1 to 10% by weight, the sugars, solubilizers, stabilizers, as well as hair conditioning and hair caring components in an amount of in each case 0.1 to 5% by weight, while the thickeners and solubilizers may be contained in this agent in an amount totaling 0.5 to 20% by weight.

Furthermore, to increase the effect, so-called swelling or penetrating materials, such as dipropylene glycol monomethyl ether, 2-pyrrolidone or imidazolidine-2-one may be contained in an amount of 1 to 30% by weight in the agent.

The ready-for-use agent for the permanent shaping of hair is obtained by mixing several components. It is most advantageous if the ready-for-use agent is prepared by mixing two components immediately (5 seconds to 5 minutes, preferable 10 seconds to 3 minutes) before use, component (A) containing the keratin-reducing active ingredient and the substrate and component (B) containing the enzyme. The individual components may be present in solid or also in liquid or thickened form. Advantageously, the agent is manufactured as a two-component package.

In a further preferred embodiment the ready-for-use agent for the permanent shaping of hair is obtained by mixing two components immediately (5 seconds to 5 minutes, preferable 10 seconds to 3 minutes) before use, component (A) containing the keratin-reducing active ingredient in an aqueous formulation and component (B) containing the enzyme and the substrate in a solid form (powder or tablet).

In a further preferred embodiment of the inventive method for the permanent shaping of hair, the latter is treated as follows: Before the application, the enzyme of component (B) is added to a solution of component (A), which contains the mercapto compound and the substrate. The hair in curlers is now wetted in a known manner with the pre-shaping agent. The original pH of the mixture of preferably about 7.5 to 10 drops during the period of action to a value of from about 6.0 to about 7.5. The period of action depends on the extent of the curling desired and on the treatment temperature. After the treatment, the hair is given a subsequent oxidative treatment and then dried.

In a further preferred embodiment of the inventive method, the hair is washed initially with a shampoo and then rinsed with water. Subsequently, the hair, which has been toweled dry, is divided into individual strands and wound onto curlers with a diameter of 5 to 30 mm and preferably of 5 to 15 mm. Since, 10 seconds to 3 minutes before the application, the enzyme of the component (B) was mixed with the component (A) containing the hair keratin-reducing mercapto compound, the substrate and preferably a buffer substance selected from ammonium hydrogen carbonate, ammonium carbonate and/or ammonium carbamate, the hair is treated with an amount, sufficient for shaping the hair, preferably of 50 to 120 g of the ready-for-use shaping agent.

After a period of action, which is sufficient for the permanent shaping of the hair and which, depending on the nature of the hair, the shaping effectiveness of the shaping agent, as well as on the application temperature, amounts to 5 to 30 minutes (10 to 30 minutes without the action of heat or 5 to 20 minutes with the action of heat), the hair is rinsed with water and then given a subsequent oxidative treatment ("fixed"). Depending on the profusion of the hair, the agent for the subsequent oxidative treatment is preferably employed in an amount of 80 to 100 g.

For the subsequent oxidative treatment of the hair on curlers or not on curlers, any subsequent treatment agent, suitable for such a treatment, can be used. Examples of oxidizing agents, which can be used in such subsequent treatment agents, are potassium and sodium bromate, sodium perborate, urea peroxide and hydrogen peroxide. The concentration of the oxidizing agent varies, depending on the application time (usually 5 to 15 minutes) and on the application temperature. Usually, the oxidizing agent is present in the aqueous subsequent oxidative treatment agent in a concentration of 0.5 to 10% by weight. The agent for the subsequent oxidative treatment may, of course, contain other materials, such as wetting agents, care materials such as cationic polymers, weak acids, buffering substances or peroxide stabilizers and exist in the form of an aqueous solution or an emulsion as well as in a thickened form on an aqueous basis, especially as a cream, a gel or a paste. These conventional additives may be contained in the subsequent treatment agent, in particular, in an amount of 0.1 to 10% by weight.

Subsequently, the curlers are removed. If necessary, after the removal of the curlers, the hair can be treated once again oxidatively. The hair is then rinsed with water, optionally laid into a water wave and subsequently dried.

A further object of the invention is a new ready-to-apply permanent hair shaping agent having an initial pH of from about 7.0 to about 14.0, preferably from about of 7.5 to about 12.0, more preferably of from about 7.5 to about 10.0, and comprising a keratin-reducing active agent, a non-ester substrate and an enzyme that catalyzes an acid-generating reaction with said non-ester substrate.

A further object of the invention is a new ready-to-apply permanent shaping agent prepared by mixing (A) an alkaline permanent shaping composition having an initial pH of from about 7.0 to about 14.0, preferably from about of 7.5 to about 12.0, more preferably of from about 7.5 to about 10.0, and comprising a keratin-reducing active agent and a non-ester substrate, 5 seconds to 3 minutes prior to use with (B) an enzyme that catalyzes an acid-generating reaction with said substrate or a composition containing said enzyme.

The agent and the method bring about an elastic, permanent and uniform shaping at the entire length from the hair regrowth (hair roots) up to the tip of the hair without producing allergic or sensitizing reactions under largely gentle conditions that do not damage the structure of the hair.

### Examples

### Example 1 Two-Component Hair Shaping Agent

| | | |
|---|---|---|
| Component A | 14.8 g | ammonium thioglycolate, 70% aqueous solution |
| | 2.6 g | ammonium hydrogen carbonate |
| | 1.2 g | 5,6-dihydrouracil |
| | 0.3 g | dimethyldiallylammonium chloride homopolymer (CTFA: polyquaternium-6) |
| | 2.0 g | glycerin polyethylene glycol ricinoleate (CTFA: PEG-35 castor oil) |
| | 1.0 g | perfume oil |
| | to pH 8.4 | ammonia |
| | ad 100.0 g | water |
| | | |
| Component B | 10.0 g | aqueous ammonium sulfate suspension of 50mg/l hydantoinase enzyme |

Before use, 100 g of component A and 1 g of component B are mixed together.

The washed, toweled dry hair, is wound onto curlers with a diameter of 6 mm and then moistened uniformly with the hair shaping agent, which is described above and was prepared one minute before use. After a period of action of 5 minutes, the initial pH of 8.4 is decreased gradually, by 0.23 units to 8,17. After a further period of action of 15 minutes at a pH of 8.17 at room temperature, the hair is rinsed thoroughly with water and then treated oxidatively with 80 g of a 3% aqueous hydrogen peroxide solution. After removal of the curlers, the hair is rinsed once more with water, laid into a water wave and subsequently dried. The hair, so treated, has a uniform and lively permanent wave.

### Example 2 Two-Component Hair Shaping Agent

| | | |
|---|---|---|
| Component A | 14.5 g | ammonium thioglycolate, 70% aqueous solution |
| | 2.0 g | thiolactic acid |
| | 2.0 g | ammonium carbonate |
| | 2.4 g | 5,6-dihydrouracil |
| | 0.7 g | vinylpyrrolidone / methacrylamidopropyl-trimethylammonium chloride copolymer (CTFA: polyquaternium-28) |
| | 1.0 g | hydrogenated, ethoxylated castor oil (CTFA: PEG-40 hydrogenated castor oil) |
| | 0.5 g | perfume oil |
| | to pH 8.48 | ammonia |
| | ad 100.0 g | water |
| | | |
| Component B | 10.0 g | aqueous ammonium sulfate suspension of 50mg/l hydantoinase enzyme |

Before use, 100 g of component A and 2 g of component B are mixed together.

The washed, toweled dry hair, is wound onto curlers with a diameter of 6 mm and then moistened uniformly with the hair shaping agent, which is described above and was prepared one minute before use. During a period of action of 5 minutes, the initial pH of 8.48 is decreased gradually, by 0.82 units to 7.66. After the whole period of action of 10 minutes at room temperature, the hair is rinsed thoroughly with water and then treated oxidatively with 80 g of a 3% aqueous hydrogen peroxide solution. After removal of the curlers, the hair is rinsed once more with water, laid into a water wave and subsequently dried. The hair, so treated, has a uniform and lively permanent wave.

### Example 3 Two-Component Hair Shaping Agent

| | | |
|---|---|---|
| Component A | 11.0 g | ammonium thioglycolate, 70% aqueous solution |
| | 7.0 g | cysteine |
| | 2.6 g | ammonium hydrogen carbonate |
| | 4.8 g | 5,6-dihydrouracil |
| | 0.7 g | vinylpyrrolidone / N,N-dimethylaminoethyl-methacrylate copolymer (CTFA: polyquaternium-11) |
| | 1.0 g | polyoxyethylene (4) lauryl ether (CTFA: laureth-4) |
| | 0.5 g | perfume oil |
| | to pH 8.48 | ammonia |
| | ad 100.0 g | water |
| | | |
| Component B | 10.0 g | aqueous ammonium sulfate suspension of 50 mg/l hydantoinase enzyme |

Before use, 100 g of component A and 2 g of component B are mixed together.

The washed, toweled dry hair, is wound onto curlers with a diameter of 6 mm and then moistened uniformly with the hair shaping agent, which is described above and was prepared one minute before use. During a period of action of 8 minutes the initial pH of 8.48 is decreased gradually by 2.04 units to 6.44. After the whole period of action of 15 minutes at room temperature, the hair is rinsed thoroughly with water and then treated oxidatively with 80 g of a 3% aqueous hydrogen peroxide solution. After removal of the curlers, the hair is rinsed once more with water, laid into a water wave and subsequently dried. The hair, so treated, has a uniform and lively permanent wave.

### Example 4 Two-Component Hair Shaping Agent

| | | |
|---|---|---|
| Component A | 11.2 g | ammonium thioglycolate, 70% aqueous solution |
| | 5.3 g | ammonium thiolactate, 50% aqueous solution |
| | 2.0 g | ammonium hydrogen carbonate |
| | 4.8 g | 5,6-dihydrouracil |
| | 1.7 g | polyoxyethylene (4) lauryl ether (CTFA: laureth-4) |
| | 1.5 g | vinylpyrrolidone / methacrylamidopropyltrimethylammonium chloride copolymer (CTFA: polyquaternium-28) |
| | 0.5 g | perfume oil |
| | to pH 8.40 | ammonia |
| | ad 100.0 g | water |
| | | |
| Component B | 10.0 g | aqueous ammonium sulfate suspension of 50mg/l hydantoinase enzyme |

Before use, 100 g of component A and 0.4 g of component B are mixed together.

The washed, toweled dry hair, is wound onto curlers with a diameter of 6 mm and then moistened uniformly with the hair shaping agent, which is described above and was prepared one minute before use. During the period of action of 30 minutes, the initial pH of 8.40 is decreased gradually, by 1.1 units to 7.30. After the period of action of 30 minutes at room temperature, the hair is rinsed thoroughly with water and then treated oxidatively with 80 g of a 3% aqueous hydrogen peroxide solution. After removal of the curlers, the hair is rinsed once more with water, laid into a water wave and subsequently dried. The hair, so treated, has a uniform and lively permanent wave.

### Example 5 Two-Component Hair Shaping Agent

| | | |
|---|---|---|
| Component A | 14.8 g | ammonium thioglycolate, 70% aqueous solution |
| | 2.0 g | ammonium hydrogen carbonate |
| | 1.1 g | hydantoin |
| | 0.6 g | vinylpyrrolidone / methyl vinyl imidazolium chloride copolymer (CTFA: polyquaternium-16) |
| | 1.9 g | coconut fatty alcohol polyoxyethylene ether (10 EO) (CTFA: coceth-10) |
| | 0.5 g | perfume oil |
| | to pH 8.2 | ammonia |
| | ad 100.0 g | water |
| | | |
| Component B | | Mixture of 1g starch powder and 0.05 mg hydantoinase enzyme (powder, lyophilisate). |

Before use component A and component B are mixed together.

The washed, toweled dry hair, is wound onto curlers with a diameter of 6 mm and then moistened uniformly with the hair shaping agent, which is described above and was prepared one minute before use. During the period of action of 30 minutes, the initial pH of 8.40 is decreased gradually, by 1.1 units to 7.30. After said period of action of 30 minutes at room temperature, the hair is rinsed thoroughly with water and then treated oxidatively with 80 g of a 3% aqueous hydrogen peroxide solution. After removal of the curlers, the hair is rinsed once more with water, laid into a water wave and subsequently dried. The hair, so treated, has a uniform and lively permanent wave.

### Example 6 Two-Component Hair Shaping Agent

| | | |
|---|---|---|
| Component A | 11.2 g | ammonium thioglycolate, 70% aqueous solution |
| | 6.0 g | ammonium thiolactate, 50% aqueous solution |
| | 2.0 g | ammonium hydrogen carbonate |
| | 2.2 g | hydantoin |
| | 0.7 g | vinylpyrrolidone / N,N-dimethylaminoethyl-methacrylate copolymer (CTFA: polyquaternium- 11) |
| | 2.0 g | polyoxyethylene (4) lauryl ether (CTFA: laureth-4) |
| | 0.5 g | perfume oil |
| | to pH 8.2 | ammonia |
| | ad 100.0 g | water |
| | | |
| Component B | 10.0 g | aqueous ammonium sulfate suspension of 50mg/l hydantoinase enzyme |

Before use, 100 g of component A and 1 g of component B are mixed together.

The washed, toweled dry hair, is wound onto curlers with a diameter of 6 mm and then moistened uniformly with the hair shaping agent, which is described above and was prepared one minute before use. During the period of action of 30 minutes, the initial pH of 8.3 is decreased gradually, by 1 unit to 7.3. After said period of action of 30 minutes at room temperature, the hair is rinsed thoroughly with water and then treated oxidatively with 80 g of a 3% aqueous hydrogen peroxide solution. After removal of the curlers, the hair is rinsed once more with water, laid into a water wave and subsequently dried. The hair, so treated, has a uniform and lively permanent wave.

### Examples 7 to 24 Two-Component Hair Shaping Agents

In a composition base according to example 6 the hydantoin was replaced by the following amounts of hydantoin derivatives respectively dihydrouracil derivatives and applied to the hair accordingly. The time dependent decrease of the pH can be seen in column 4 and 5 below.

| **example** | **substrate** | **amount** | **pH initially** | **pH end** | **treating time** |
|---|---|---|---|---|---|
| 7 | 5-hydroxymethyl-hydantoin | 1.5 g | 8.3 | 7.8 | 30 min |
| 8 | 5-hydroxymethyl-hydantoin | 3.0 g | 8.3 | 7.2 | 25 min |
| 9 | 5-hydroxymethyl-hydantoin | 6.0 g | 8.3 | 6.5 | 20 min |
| 10 | 5-(2-hydroxyethyl)-hydantoin | 1.6 g | 8.3 | 7.3 | 30 min |
| 11 | 5-(2-hydroxyethyl)-hydantoin | 3.2 g | 8.3 | 7.0 | 27 min |
| 12 | 5-(2-hydroxyethyl)-hydantoin | 6.4 g | 8.3 | 6.5 | 20 min |
| 13 | 5-(2-carboxyethyl)-hydantoin | 1.8 g | 8.3 | 7.3 | 30 min |
| 14 | 5-(2-carboxyethyl)-hydantoin | 3.6 g | 8.3 | 6.8 | 25 min |
| 15 | 5-(2-carboxyethyl)-hydantoin | 7.2 g | 8.3 | 6.4 | 20 min |
| 16 | 5-(2-methoxyethyl)-hydantoin | 1.6 g | 8.3 | 7.3 | 30 min |
| 17 | 5-(2-methoxyethyl)-hydantoin | 3.2 g | 8.3 | 7.1 | 25 min |
| 18 | 5-(2-methoxyethyl)-hydantoin | 6.4 g | 8.3 | 6.5 | 20 min |
| 19 | 5-(2-dimethylaminoethyl)-hydantoin | 2.0 g | 8.3 | 7.1 | 30 min |
| 20 | 5-(2-dimethylaminoethyl)-hydantoin | 4.0 g | 8.3 | 6.9 | 25 min |
| 21 | 5-(2-dimethylaminoethyl)-hydantoin | 6.0 g | 8.3 | 6.7 | 20 min |
| 22 | 5-(2-hydroxyethyl)-dihydrouracil | 1.8 g | 8.3 | 7.2 | 30 min |
| 23 | 5-(2-hydroxyethyl)-dihydrouracil | 3.6 g | 8.3 | 6.8 | 25 min |
| 24 | 5-(2-hydroxyethyl)-dihydrouracil | 7.2 g | 8.3 | 6.4 | 20 min |

## Claims

1. A cosmetic composition comprising at least one cosmetic ingredient and an enzyme that catalyzes an acid-generating reaction with a non-ester substrate.

2. The composition of claim 1, further comprising a non-ester substrate for that enzyme.

3. The composition of claim 2, in which said non-ester substrate is selected from the group consisting of compounds represented by formulas (I) and (II) 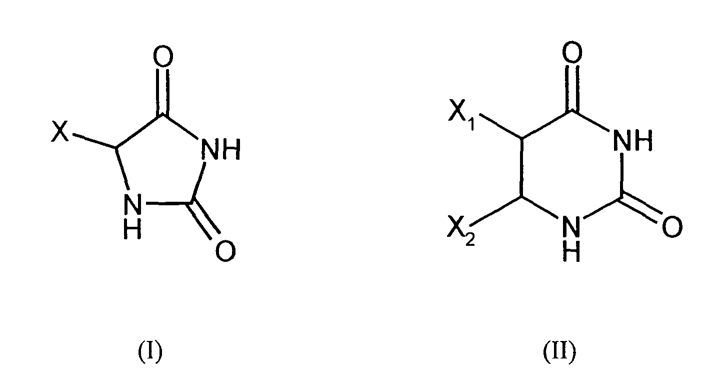 wherein X, X₁ and X₂ are independently selected from the group consisting of hydrogen, alkyl, aryl, arylalkyl and heterocyclic.

4. The composition of claim 1, wherein the cosmetic ingredient is selected from the group consisting of thickening agents, such as bentonite, fatty acids, starch, polyacrylic acid and its derivatives, cellulose derivatives, alginates, Vaseline, paraffin oils, wetting agents or emulsifiers from the classes of anionic, cationic, amphoteric or nonionic surface-active substances, such as fatty alcohol sulfates, fatty alcohol ether sulfates, alkylsulfonates, alkylbenzenesulfates, quaternary ammonium salts, alkylbetaines, ethoxylated alkylphenols, fatty acid alkanolamides or ethoxylated fatty esters, furthermore opacifiers, such as polyethylene glycol esters, alcohols, such as ethanol, propanol, isopropanol, polyols, such as ethylene glycol, 1,2- or 1,3-dihydroxy-propane, 1,2-, 1,3- or 1,4-dihydroxy-butane, 1,2-, 1,3-, 1,4- or 1,5-dihydroxy-pentane and glycerin, sugars, such as D-glucose, solubilizers, stabilizers, buffering substances, perfume oils, dyes as well as hair conditioning and hair careing components, such as cationic polymers, silicone polymers, cationic silicone polymers, lanolin, lanolin derivatives, cholesterol, pantothenic acid and betaine.

5. A method for time dependent decrease of pH in a cosmetic composition for treating skin or hair, said method comprising the steps of:
(i) adding an enzyme that catalyzes an acid-generating reaction with a non-ester substrate to an alkaline composition having an initial pH of from about 7.0 to about 14 and comprising said non-ester substrate to form a ready-to-apply cosmetic composition;
(ii) treating the skin or hair of a person with said ready-to-apply cosmetic composition for a time sufficient to achieve the desired cosmetic effect.

6. The method as defined in claim 5, wherein the enzyme and the substrate is selected so that the initial pH of from about 7.0 to about 12 is lowered within a time interval of from 5 to 40 minutes by at least 0.5 pH units.

7. A method for permanently shaping hair, said method comprising the steps of:
(a) adding an enzyme that catalyzes an acid-generating reaction with a non-ester substrate to an alkaline permanent shaping composition having an initial pH of 7.0 to 14 and comprising a keratin-reducing active agent and a non-ester substrate immediately prior to treating the hair with said shaping agent, to form a ready-to-apply alkaline shaping agent;
(b) putting the hair in a desired shape;
(c) treating the hair with said ready-to-apply shaping agent for a period of action sufficient for permanent shaping of hair;
(d) after the period of action of said shaping agent, optionally rinsing the hair with water;
(e) after rinsing of the hair in step (d) oxidatively treating the hair and then optionally again rinsing the hair with water; and
(f) subsequently optionally putting the hair in a water wave and drying the hair.

8. The method as defined in claim 7, wherein the enzyme and the non-ester substrate are selected so that the initial pH of 7.0 to 12 is lowered within a time interval of from 5 to 40 minutes by at least 0.5 pH units.

9. The method as defined in claim 7, wherein said enzyme and said non-ester substrate is selected so that said initial pH of 7.5 to 10 is lowered within from 10 to 30 minutes by about 0.5 to about 3.0 pH units.

10. The method as defined in claim 7, 8 or 9, wherein said enzyme is hydantoinase.

11. The method as defined in claim 7, 8, 9 or 10, wherein said non-ester substrate is selected from a hydantoin and a dihydrouracil as represented in formulas (I) and (II).

12. The method as defined in claim 7, 8, 9, 10 or 11, wherein said initial pH is from 7.8 to 9.5.

13. The method as defined in claim 7, 8, 9, 10, 11 or 12, wherein said initial pH is from 8.0 to 9.0.

14. The method as defined in claim 7, 8, 9, 10, 11, 12 or 13, wherein the enzyme is added to said alkaline permanent shaping composition 10 seconds to 3 minutes prior to use of the ready-to-apply cosmetic composition.

15. A ready-to-apply permanent hair shaping agent prepared by mixing (A) an alkaline permanent shaping composition having an initial pH of 7.0 to 14 and comprising a keratin-reducing active agent and a non-ester substrate, 5 seconds to 3 minutes prior to use with (B) an enzyme that catalyzes an acid-generating reaction with said non-ester substrate or a composition comprising said enzyme.

16. A ready-to-apply permanent hair shaping agent as defined in claim 15, wherein said initial pH is from about 7.0 to about 12.0.

17. A ready-to-apply permanent hair shaping agent as defined in claim 15, wherein said initial pH is from about 7.5 to about 10.0.

18. A ready-to-apply permanent hair shaping agent as defined in claim 15, wherein said initial pH is from about 8.0 to about 9.0.

19. A ready-to-apply permanent hair shaping agent as defined in claim 15, wherein said enzyme is hydantoinase.

20. A ready-to-apply permanent hair shaping agent as defined in claim 15, wherein the concentration of the enzyme in the shaping agent is 0.01 to 0.50 % by weight.

21. A ready-to-apply permanent hair shaping agent as defined in claim 15, wherein said non-ester substrate is selected from hydantoin and dihydrouracil.

22. A ready-to-apply permanent hair shaping agent as defined in claim 15, wherein the concentration of the non-ester substrate in the agent is 1 to 10 % by weight.

23. A ready-to-apply permanent hair shaping agent as defined in claim 15, wherein the keratin-reducing active agent is selected from mercaptanes.

24. A ready-to-apply permanent shaping agent as defined in claim 23, wherein the keratin-reducing active agent is selected from thioglycolic acid, thioglycerine, thioglycolamide, thiolactic acid, 3-mercaptoprpionic acid, cystein, cysteamine, homocysteine, alkyl or acyl cysteins, or the salts of these compounds.

25. A ready-to-apply permanent hair shaping agent as defined in claim 23 or 24, wherein the keratin-reducing active agent is present an amount of 5 to 15 % by weight.

26. A ready-to-apply permanent hair shaping agent having an pH of from about 7.0 to about 14.0 and comprising a keratin-reducing active agent, a non-ester substrate and an enzyme that catalyzes an acid-generating reaction with said non-ester substrate.

27. A ready-to-apply permanent hair shaping agent having an pH of from about 7.5 to about 12.0 and comprising a keratin-reducing active agent, a non-ester substrate and an enzyme that catalyzes an acid-generating reaction with said non-ester substrate.

28. The composition, agent or method of claims 1, 5, 7, 15, 26 or 27, in which said enzyme has the property that it shows a half-life in the presence of 10% by weight thioglycolate at pH 8.5 of greater than 30 min.
